# EUROPEAN PATENT APPLICATION

(11) **EP 2 042 478 A1**
(43) Date of publication of application: **01.04.2009**
(21) Application number: 07790747.5
(22) Date of filing: 13.07.2007
(51) Int. Cl.: C07C 45/68, C07C 49/213, C07C 49/255, C07C 49/35, C07C 67/343, C07C 69/734, C07C 221/00, C07C 225/22, C07D 213/55, C07D 317/60, C07D 319/10, C07D 405/06, C07B 53/00, C07B 61/00

(54) **METHOD FOR PRODUCING -DIARYL ELECTRON-WITHDRAWING GROUP SUBSTITUTED COMPOUND**

(30) Priority: 19.07.2006 JP 2006196491
(71) Applicant: Japan Science and Technology Agency, Kawaguchi-shi Saitama 332-0012 (JP); National University Corporation Hokkaido University, Sapporo-shi Hokkaido 060-0808 (JP); BANYU PHARMACEUTICAL CO., LTD., Tokyo 102-8667 (JP)
(72) Inventor: MIYAURA, Norio, Sapporo-shi Hokkaido 060-8628 (JP); YAMAMOTO,Yasunori, Sapporo-shi Hokkaido 060-8589 (JP); NISHIKATA, Takashi, Sapporo-shi Hokkaido 060-0819 (JP); ITOH, Takahiro, Tsukuba-shi Ibaraki 300-2611 (JP)
(74) Representative: Prüfer & Partner GbR European Patent Attorneys
(86) International application number: PCT/JP2007/063966
(87) International publication number: WO 2008/010455

(57) **Abstract**

An electron withdrawing group substituted β-arylolefin derivative represented by general formula (I) 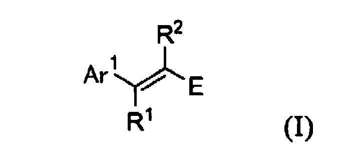 (in the formula, Ar¹ represents an aryl group, E represents a formyl, acyl and so on) is allowed to react with an arylboronic acid represented by general formula (II) Ar²-BXₘMₙ in the general formula (IV) RhYₒL¹ₚ (Chiraphos)_{q} (in the formula, Y represents ClO₄, BF₄, PF₆, SbF₆, OTf, halogen atom, hydroxyl group, alkoxy group or acyloxy group, L¹ represents an organic ligand) to produce an optically active β-diaryl electron withdrawing group substituted compound represented by general formula (V)

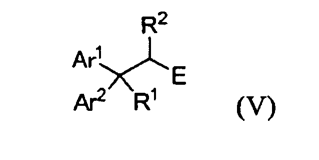

## Description

### Field of the Invention

This invention relates to a method for manufacturing a β-diaryl electron withdrawing group substituted compound.

### Background of the Invention

An optically active β-diaryl electron withdrawing group substituted compound is a useful compound used as an intermediate in pharmaceutical and agriculture chemicals and the like, and a method that can produce the compound in high yield with excellent selectivity is urgently needed.
Methods previously published involved the use of rhodium-optically active BINAP complex catalysts and optically active β-aryl esters and β-arylamides were produced. (See Patent References 1-4.) However, when rhodium-optically active BINAP complex catalysts were used, the optical purity of the optically active β-diaryl electron withdrawing group substituted compound was not necessarily adequate.

[Patent Reference 1] publication before examination
   Japanese Unexamined Patent Publication (Kokai) 2001-64233
[Patent Reference 2]
   Japanese Unexamined Patent Publication (Kokai) 2001-131125
[Patent Reference 3]
   Japanese Unexamined Patent Publication (Kokai) 2003-160549
[Patent Reference 4]
   Japanese Unexamined Patent Publication (Kokai) 2004-315396

### Problems to be solved by the Invention

The inventors conducted an extensive study of the methods that manufacture a β-diaryl electron withdrawing group substituted compound in excellent yields and high selectivity according to the conventional technology described above. As a result, the inventors discovered the benefits of specific rhodium complex catalysts.
That is, the objective of the present invention is to present a method that can produce an optically active β-diaryl electron withdrawing group substituted compound in excellent yield with excellent selectivity.

### Means to solve the Problems

In order to solve the problem, the method for manufacturing a β-diaryl electron withdrawing group substituted compound of the present invention includes an electron withdrawing group substituted β-arylolefin derivative represented by general formula (I) 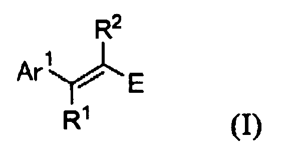 (in the formula, Ar¹ represents a substituted or unsubstituted aryl group, R¹ and R² individually may be identical or different from each other and represent hydrogen or alkyl groups and E represents a formyl, acyl, carboxyl, alkoxycarbonyl, carbamoyl, cyano or nitro group) is allowed to react with an arylboronic acid or its derivative represented by general formula (II) Ar²-BXₘMₙ or an arylboronic acid anhydride represented by general formula (III) 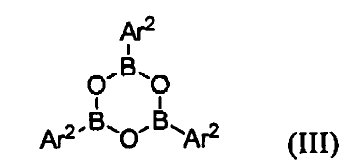 (in formulae II and III, Ar² represents a substituted or unsubstituted aryl group, X indicates a hydroxyl group, alkoxy group or fluorine atom, m is an integer from one to three, M is an alkali metal and n is an integer, zero or one) in the presence of a rhodium complex catalyst represented by general formula (IV) RhYₒL¹ₚ (Chiraphos)_{q} (in the formula, Y represents ClO₄, BF₄, PF₆, SbF₆, OTf, halogen atom, hydroxyl group, alkoxy group or acyloxy group, L¹ represents an organic ligand, o, p, and q each represent an integer from zero to six) to produce an optically active β-diaryl electron withdrawing group substituted compound represented by general formula (V) 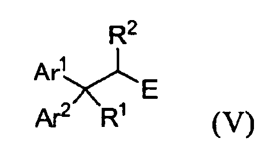 (in formula V, Ar¹, Ar², R¹, R² and E are identical to the substituents used in formulae I-IV).

The addition of a base to the reaction system is preferred.
The base is preferably one or at least two selected from a group containing amines, alkali metal hydroxides or their salts of weak acids, alkaline earth metal hydroxides or their salts of weak acids and quaternary ammonium hydroxides or their salts of weak acids.
The optically active β-diaryl electron withdrawing group substituted compound preferably containing nitrogen or an optically active β-diaryl ester.

### Advantageous effect of the Invention

According to this invention, an optically active β-diaryl electron withdrawing group substituted compound can be obtained in excellent yield and selectivity.

### Detailed Description of the Invention

The present invention is a method wherein (1) an electron withdrawing group substituted olefin derivative, (2) an arylboronic acid or its derivative or an arylboronic acid anhydride and (3) a rhodium complex catalyst as described below are allowed to react to produce (4) a β-diaryl electron withdrawing group substituted compound.

### <β-diaryl electron withdrawing group substituted compound >

An electron withdrawing group substituted β-arylolefin derivative as a substrate (an α,β-unsaturated β-arylcarbonyl compound) is represented by the general formula (I) 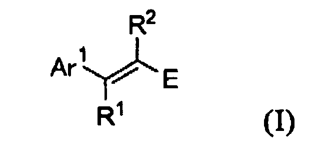 (in the formula, Ar¹ represents a substituted or unsubstituted aryl group, R¹ and R² individually may be identical or different from each other and represent hydrogen or alkyl groups and E represents a formyl, acyl, carboxyl, alkoxycarbonyl, carbamoyl, cyano or nitro group).

Aromatic rings such as phenyl, naphthyl, pyrrolyl, thiophenyl, furanyl, indolyl, benzofuranyl, pyridyl and the like may be cited as Ar¹. As the substituents for the aromatic rings, chlorine atom, fluorine atom, hydroxyl group, alkyl groups containing one to eight carbon atoms, phenyl groups that may also contain alkyl groups with one to eight carbon atoms, alkenyl groups with two to eight carbon atoms, alkynyl groups with two to eight carbon atoms, alkoxy groups with one to eight carbon atoms, alkylthio groups with one to eight carbon atoms, cyano groups, formyl groups, acyl groups with two to eight carbon atoms, benzoyl groups that may also contain alkyl groups with one to eight carbon atoms, alkoxycarbonyl groups with two to eight carbon atoms, phenoxycarbonyl groups that may also contain alkyl groups with one to eight carbon atoms, amino groups that may also contain alkyl groups with one to eight carbon atoms, carbamoyl groups that may also contain alkyl groups with one to eight carbon atoms, nitro groups, fluoroalkyl groups with one to eight carbon atoms, phenoxy groups that may also contain alkyl groups with one to eight carbon atoms, cyclohexyloxy groups and the like may be cited. More specifically, a phenyl group, o-tolyl group, m-tolyl group, p-tolyl group, o-methoxyphenyl group, m-methoxyphenyl group, p-methoxyphenyl group, p-trifluoromethyl phenyl group, 2-naphthyl group, 3-pyridyl group, 2-pyridyl group, 3-[6-(N-benzyl-N-isopropylamino-2-butoxycarbonyl-3-pyridyl group, (2-methoxycarbonyl-4-propoxyphenyl group and the like, for example, may be cited as Ar¹.

Hydrogen or alkyl groups with one to eight carbon atoms may be cited as R¹ and R². A methyl group, an ethyl group, a n-propyl group, a isopropyl group, a n-pentyl group, a cyclohexyl group and the like, for example, may be cited as the alkyl group. Hydrogen is preferably used as R¹ and R².

A formyl group, an acyl group with two to eight carbon atoms, an alkoxycarbonyl group with two to eight carbon atoms, a cyano group, a carbamoyl group that may also contain alkyl groups with one to eight carbon atoms and a nitro group, for example, may be cited as E.
Methyl carbonyl, ethyl carbonyl, n-propylcarbonyl, t-butylcarbonyl, n-pentylcarbonyl, n-hexylcarbonyl, cyclohexyl carbonyl, phenylcarbonyl and benzylcarbonyl groups, for example, may be cited as the acyl group with two to eight carbon atoms.
Alkyloxy, phenyloxy and benzyloxy groups, for example, may be cited as the alkoxy in the alkoxycarbonyl group with two to eight carbon atoms. Therefore, methoxycarbonyl, ethoxycarbonyl, n-propoxycarbonyl, iso-propoxycarbonyl, n-butoxycarbonyl, s-butoxycarbonyl, t-butoxycarbonyl, n-pentoxycarbonyl, n-hexyloxycarbonyl, cyclohexyloxycarbonyl phenoxycarbonyl, benzyloxycarbonyl groups and the like, for example, may be cited as the alkoxycarbonyl group described above.
As the carbamoyl group that may also contain alkyl groups with one to eight carbon atoms, N-methylcarbamoyl, N,N-dimethylcarbamoyl, N-ethylcarbamoyl, N,N-diethylcarbamoyl, N-n-propylcarbamoyl, N,N-di-n-propylcarbamoyl, N-isopropylcarbamoyl, N,N-di-iso-propylcarbamoyl, N-n-butylcarbamoyl, N,N-di-n-butylcarbamoyl, N-s-butylcarbamoyl, N,N-di-s-butylcarbamoyl, N-t-butylcarbamoyl, N,N-di-t-butylcarbamoyl, N-n-pentylcarbamoyl, N,N-di-n-pentylcarbamoyl, N-n-hexylcarbamoyl, N,N-di-n-hexylcarbamoyl, N-cyclohexylcarbamoyl, N,N-dicyclohexylcarbamoyl groups and the like, for example, may be cited.

As specific examples of the compound indicated by the general formula (I) shown above, trans-styrylmethylketone, trans-chalcone, trans-4-(2-naphthyl)-3-butene-2-one, trans-methylcinnamate, isopropyl cinnamate, t-butylcinnamate, trans-t-butyl-p-tolyl acrylate, trans-t-butyl-(4-methoxyphenyl) acrylate, trans-t-butyl-(2-methoxyphenyl) acrylate, trans-t-butyl-(4-trifluoromethylphenyl) acrylate, trans-t-butyl-(2-naphthyl) acrylate, trans-t-butyl-(3-pyridyl) acrylate and the like, for example may be cited.

### <Arylboronic acid or its derivatives or arylboronic acid anhydride>

The arylboronic acid or its derivatives (II) or arylboronic acid anhydride (III) undergo asymmetric conjugated addition reactions with the reactive substrates described above and are independently represented by general formula (II) Ar²-BXₘMₙ or general formula (III). 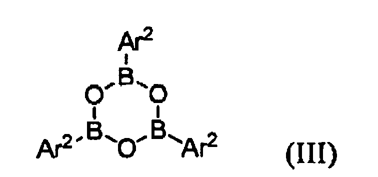 (In formulae II and III, Ar² represents a substituted or unsubstituted aryl group, X indicates a hydroxyl group, alkoxy group or fluorine atom, m is an integer from one to three, M is an alkali metal and n is an integer zero or one.)

Aromatic rings such as phenyl group, naphthyl group, pyrrolyl group, thiophenyl group, furanyl group, indolyl group, benzofuranyl group, pyridyl group and the like may be cited as Ar². As the substituents for these aromatic rings, chlorine atoms, fluorine atoms, alkyl groups with one to eight carbon atoms, phenyl groups that may also contain alkyl groups with one to eight carbon atoms, alkenyl groups with two to eight carbon atoms, alkynyl groups with two to eight carbon atoms, alkoxy groups with one to eight carbon atoms, alkylthio groups with one to eight carbon atoms, cyano groups, formyl groups, acyl groups with two to eight carbon atoms, benzoyl groups that may also contain alkyl groups with one to eight carbon atoms, alkoxycarbonyl groups with two to eight carbon atoms, phenoxycarbonyl groups that may also contain alkyl groups with one to eight carbon atoms, amino groups that may also contain alkyl groups with one to eight carbon atoms, carbamoyl groups that may also contain alkyl groups with one to eight carbon atoms, nitro groups, fluoroalkyl groups with one to eight carbon atoms, phenoxy groups that may also contain alkyl groups with one to eight carbon atoms, cyclohexyloxy groups and the like may be cited.

When an alkoxy group is used as the X, alkoxy groups containing alkyl groups with one to eight carbon atoms, phenoxy groups that may also contain alkyl groups with one to eight carbon atoms, the cyclohexyloxy group or a compound represented by a, b, c or d of the formulae (VI) below (in the formula VI, r represents an integer from one to four, s and t independently represent an integer from zero to five and Me represents a methyl group) may, for example, be cited as the alkoxy group.

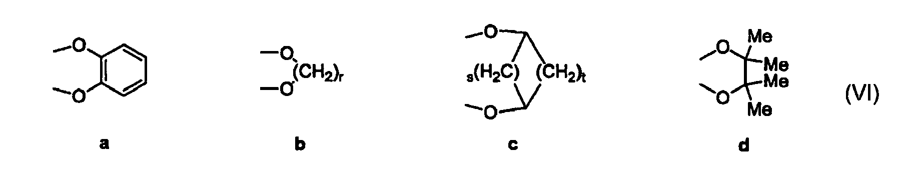

Potassium, sodium and the like may be cited as the M.

As specific examples of the arylboronic acid indicated by the general formula (II), phenyl boronic acid, o-tolylboronic acid, m-tolylboronic acid, p-tolylboronic acid, o-methoxyphenyl boronic acid, m-methoxyphenyl boronic acid, p-methoxyphenyl boronic acid, 3,4-dimethoxyphenyl boronic acid, 3,4-methylene dioxyphenyl boronic acid, 4-bromo-3-fluorophenyl boronic acid, 4-(N,N-dimethyl) aminophenyl boronic acid, 1-naphthyl boronic acid, 2-naphthyl boronic acid, 2,3-dihydrobenzofuranyl boronic acid, 2-pyrrolyl boronic acid, 2-thiophenyl boronic acid and 2-furanylboronic acid may be cited. In addition, as the derivatives of the arylboronic acid of formula (II), trifluoropotassium salts of the boronic acids mentioned above may be listed as examples. As specific examples of formula (III), anhydrides of arylboronic acids mentioned above may be cited. The amount of the arylboronic acid or its derivatives or arylboronic acid anhydride added may be at a mole ratio of from about 0.1 to fifty per α,β-unsaturated-β-aryl carbonyl compound (I), but a mole ratio range from one to twenty is preferred.

### <Rhodium complex catalyst>

The rhodium complex catalyst is represented by general formula (IV) RhYₒL¹ₚ (Chiraphos)_{q}. (In the formula IV, Y represents ClO₄, BF₄, PF₆, SbF₆, OTf, halogen atom, hydroxyl group, alkoxy group or acyloxy group, and L¹ indicates an organic ligand. Integers from zero to six are individually represented by o, p and q.)

As specific examples of Y, fluorine atom, chlorine atom, bromine atom, iodine atom, ClO₄, BF₄, PF₆ and SbF₆ anions and the like may be cited. When an organic compound containing oxygen is used as the Y, the hydroxyl group, an alkoxy group containing alkyl groups with one to eight carbon atoms, the acetyl acetonate group, the trifluoromethane sulfonate group, the acetoxy group and the like may be cited.
As the Y, ClO₄, BF₄, PF₆ and SbF₆ anions are preferred, and BF₄ anion is particularly preferred. In addition, o in formula IV is preferably 1.

As the organic ligand L¹, CO, NO, NH₂ and NH₃ as well as olefins, acetylenes, aromatic compounds, organic compounds containing oxygen, organic compounds containing nitrogen, organic compounds containing sulfur and the like may be cited.
As the olefin compounds, ethylene, allyls, butadiene, cyclohexene, 1,3-cyclohexadiene, cyclooctene (coe), 1,5-cyclooctadiene (cod), norbornadiene (nbd), ethyl acrylate, methacrylate esters, cyclopentadienyls, pentamethyl cyclopentadienyls and the like may be listed as examples.
As the acetylene compounds, acetylene, 1,2-dimethylacetylene, 1,4-pentadiene, 1,2-diphenylacetylene and the like may be listed as examples. As the aromatic compound, benzene, p-cymene, mesitylene, hexamethylbenzene, naphthalene and anthracene may be listed as examples. As the organic compound containing oxygen, acetates, benzoates, acetyl acetonates, trifluoromethane sulfonate and the like may be listed as examples. As the organic compound containing nitrogen, acetonitrile, benzonitrile, t-butyl-isocyanide, pyridine, 1,10-phenanthroline and 2,2'-bipyridyl may be listed as examples. As the organic compound containing sulfur, dimethyl sulfoxide, dimethyl sulfide, thiophene, carbon disulfide, carbon sulfide, thiophenol and the like may be listed as examples.

L¹ is preferably carbon monoxide, an olefin such as ethylene, cyclooctene (coe), norbornadiene (nbd), 1,5-cyclooctadiene (cod) and the like, or acetonitrile. L¹ is more preferably an olefin such as ethylene, cyclooctene, norbonadiene, 1,5-cyclooctadiene and the like, particularly preferably norbornadiene and most preferably 1,5-norbornadiene. In addition p in formula IV is preferably one.

Chiraphos [(2S, 3S)-(-)-bis(diphenylphosphino) butane and (2R,3R)-(+)-bis(diphenylphosphino) butane are optically active phosphine ligands and are used in asymmetric synthesis using a transition metal. They are phosphines containing two phosphorus atoms and are ligands. Chiraphos includes (S,S)-Chiraphos and (R,R)-Chiraphos.
In formula IV q is preferably one.

The rhodium complex catalyst (IV) is obtained, for example, by mixing a rhodium complex such as [Rh(nbd)₂]BF₄ and Chiraphos. The L in formula (IV) can be altered by changing the nbd in the rhodium complex described above to another organic ligand. The Y in formula (IV) can be altered by changing BF₄ in the rhodium complex described above to another salt. A rhodium complex catalyst obtained in the manner described may be introduced into a reaction system in the present invention, or the rhodium complex catalyst may be formed in the reaction system by adding the individual components described above to the reaction system. The amount of Chiraphos in the rhodium complex catalyst described above varies according to the reaction conditions, but the amount present may be at a mole ratio of from 0.1 to about fifty based on the elemental rhodium (Rh in the rhodium complex catalyst) co-present in the reaction system. A mole ratio range from 0.9 to about five is preferred.
In addition, the amount of the rhodium complex catalyst indicated by the (IV) described above may be at a mole ratio of from 0.00001 to about 0.5 based on the reaction substrate (the compound of formula I), and the mole ratio range of from 0.0001 to about 0.05 is preferred.

A monovalent cationic is preferred as the valence for the Rh in the rhodium complex catalyst (IV).

### <Reaction conditions>

A solvent is ordinarily used in the present invention. As the solvent, those that can dissolve the reaction starting materials and catalyst system are preferably used. As specific examples of the solvent, aromatic solvents such as toluene, xylene and the like, aliphatic solvents such as pentane, hexane and the like, halogenated hydrocarbon solvents such as methylene chloride and the like, ether solvents such as ether, tetrahydrofuran, 1,4-dioxane and the like, alcohol type solvents such as methanol, ethanol, 2-propanol, butanol, benzyl alcohol and the like, organic solvents containing hetero atoms such as acetonitrile, N,N-dimethyl formamide, N-methylpyrrolidone, pyridine, dimethyl sulfoxide and the like and water, for example, may be used. These solvents may be used individually or as a mixture of at least two of them in optional proportions.
The reaction temperature in the present invention may ordinarily be in the range of from -40°C to about 200°C, but from 50°C to 120°C is preferably used. In addition, the reaction is preferably conducted in an atmosphere of an inert gas such as nitrogen gas, argon gas and the like in order to prevent the deactivation of the catalyst by oxygen during the reaction. The pressure during the reaction is not particularly restricted, but the reaction is ordinarily conducted at atmospheric pressure. A reaction temperature of about 20°C to 80°C is best.
The reaction time varies according to conditions such as reaction substrate concentration, temperature and the like, but the reaction is ordinarily completed in several minutes to about thirty hours.
The reaction mode of the present invention may be batch type or continuous.

### <Base>

In the present invention, base is preferably added to the reaction system. As the base, one or at least two selected from the group containing amines, alkali metal hydroxides or their salts with weak acids, alkaline earth metal hydroxides or their salts with weak acids and quaternary ammonium hydroxides or their salts with weak acids may be cited. As the base, one or at least two selected from lithium hydroxide, sodium hydroxide, potassium hydroxide, sodium carbonate, sodium bicarbonate, potassium carbonate, potassium bicarbonate, cesium carbonate and tri-potassium phosphate are preferred.
The amount of the base added may be at a mole ratio of from 0.0001 to five based on arylboronic acid or its derivatives or arylboronic acid anhydride, and a mole ratio of from 0.001 to three is more preferred.

### <Product>

An optically active β-diaryl electron withdrawing group substituted compound is obtained as the product from the reaction substrate described above using general formula (I). As specific examples of the optically active β-diaryl electron withdrawing group substituted compound, (S)-4-(4-methoxyphenyl)-4-phenyl-2-butanone, (S)-4-(3,4-methylene dioxyphenyl)-4-phenyl-2-butanone, (S)-4-(3-methoxyphenyl)-4-(2-naphthyl)-2-butanone, (S)-3-(3-methoxyphenyl)-1,3-diphenyl propanon-1-one, (S)-3-(3-methoxyphenyl)-1-(4-methoxyphenyl)-3-phenylpropane-1-one, (S)-tert-butyl-3-(4-methoxyphenyl)-3-phenyl propanoate, (S)-tert-butyl-3-(3-methoxyphenyl)-3-phenyl propanoate, (S)-tert-butyl-3-(3,4-dimethoxyphenyl)-3-phenyl propanoate, (S)-tert-butyl-3-(3,4-methylene dioxyphenyl)-3-phenyl propanoate, (S)-tert-butyl-3-(4-(N,N-dimethylamino)phenyl)-3-phenyl propanoate, (S)-tert-butyl-3-(3-methoxyphenyl)-3-(4-methoxyphenyl) propanoate, (R)-tert-butyl-(2-methoxyphenyl)-3-(3-methoxyphenyl) propanoate, (S)-tert-butyl-3-(3-methoxyphenyl)-3-(p-tolyl) propanoate, (S)-tert-butyl-3-(3-methoxyphenyl)-3-(4-trifluoromethylphenyl) propanoate, (S)-tert-butyl-3-(3-methoxyphenyl)-3-(2-naphthyl) propanoate, (R)-tert-butyl-3-(3-methoxyphenyl)-3-(3-pyridyl) propanoate, methyl 2-(1-(3,4-methylene dioxyphenyl)-2-(tert-butoxycarbonyl) ethyl-5-propoxy benzoate, tert-butyl-(S)-3-[6-(benzyl)(isopropyl) amino]-2-butoxycarbonyl-3-pyridinyl)-3-phenyl propanoate, tert-butyl-(S)-3-[6-(benzyl)(isopropyl) amino]-2-butoxycarbonyl-3-pyridinyl]-3-(4-methoxyphenyl) propanoate, tert-butyl-(S)-3-[6-(benzyl)(isopropyl) amino]-2-butoxycarbonyl-3-pyridinyl]-3-(4-bromo-3-fluorophenyl) propanoate, tert-butyl-(S)-3-[6-(benzyl)(isopropyl) amino]-2-butoxycarbonyl-3-pyridinyl]-3-(3,4-methylenedioxyphenyl) propanoate, tert-butyl-(S)-3-[6-(benzyl)(isopropyl) amino]-2-butoxycarbonyl-3-pyridinyl]-3-(2,3-dihydro-1-benzofuran-6-yl) propanoate and their mirror image isomers may be cited.

Now, intermediates for optically active pharmaceutical synthesis such as the intermediate for an optically active endothelin receptor antagonist agent (formulae VIII and IX), an intermediate for PDE4 inhibitor, CDP-840 (formula X), an intermediate for the muscarinic receptor antagonist, tolterodine, and the like can be obtained in excellent optical purity by applying the present invention.

The present invention will be explained in further detail below, but the present invention is not restricted by the examples.

### [Example 1]

The individual products 4a-4p listed in the Table 1 below were prepared as optically active β-diaryl electron withdrawing group substituted compound. Reaction formula of products 4(4a-4p)is shown below. In formula VII, the symbol 1 indicates a reaction substrate, symbol 2 indicates an arylboronic acid and FG represents a substituent. Symbol 3a indicates a rhodium complex catalyst, and symbol 4 indicates a product.

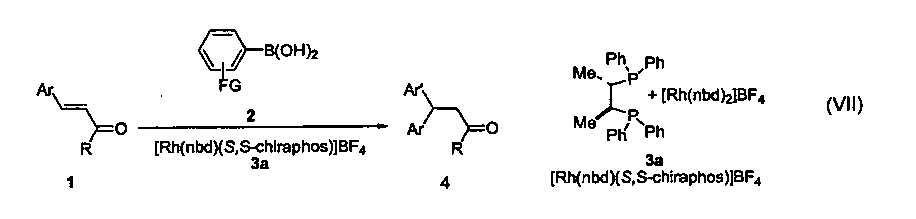

**[Table 1]**

| Exp. No. | Compound 1 | | Compound 2 | Base | Temp./time (°C/hour) | Product | Yield %^{b} | %ee^{c} |
|---|---|---|---|---|---|---|---|---|
| | Ar = | R = | FG = | | | | | |
| 1 | Ph | Me | 4-MeO | - | 20/5 | 4a | 0 | -- |
| 2 | Ph | Me | 4-MeO | Et₃N | 20/5 | 4a | 0 | -- |
| 3 | Ph | Me | 4-MeO | KHCO₃ | 20/5 | 4a | 0 | -- |
| 4 | Ph | Me | 4-MeO | K₂CO₃ | 20/5 | 4a | 96 | 84 |
| 5 | Ph | Me | 4-MeO | KOH | 20/5 | 4a | 99 | 84 |
| 6 | Ph | Me | 3,4-(CH₂O₂)^{d} | K₂CO₃ | 20/5 | 4b | 99 | 89 |
| 7 | 2-naphthyl | Me | 3-MeO | K₂CO₃ | 20/5 | 4c | 95 | 83 |
| 8 | Ph | Ph | 3-MeO | K₂CO₃ | 20/5 | 4d | 99 | 86 |
| 9 | Ph | 4-MeOC₆H₄ | 3-MeO | K₂CO₃ | 20/5 | 4e | 99 | 83 |
| 10 | Ph | O-t-Bu | 4-MeO | Et₃N | 65/21 | 4f | 40 | 91 |
| 11 | Ph | O-t-Bu | 4-MeO | K₂CO₃ | 65/21 | 4f | 76 | 91 |
| 12 | Ph | O-t-Bu | 4-MeO | Cs₂CO₃ | 65/21 | 4f | 83 | 92 |
| 13 | Ph | O-t-Bu | 4-MeO | KOH | 50/6 | 4f | 92 | 93 |
| 14 | Ph | O-t-Bu | 3-MeO | KOH | 50/6 | 4g | 95 | 90 |
| 15 | Ph | O-t-Bu | 3,4-(MeO)₂ | KOH | 50/6 | 4h | 88 | 88 |
| 16 | Ph | O-t-Bu | 3,4-(CH₂O₂)^{d} | KOH | 50/6 | 4i | 94 | 94 |
| 17 | Ph | O-t-Bu | 4-Me₂N | KOH | 50/6 | 4j | 59 | 82 |
| 18 | 4-MeOC₆H₄ | O-t-Bu | 3-MeO | KOH | 80/6 | 4k | 97 | 90 |
| 19 | 2-MeOC₆H₄ | O-t-Bu | 3-MeO | KOH | 80/6 | 4l | 96 | 91 |
| 20 | 4-MeC₅H₄ | O-t-Bu | 3-MeO | KOH | 80/6 | 4m | 92 | 91 |
| 21 | 4-CF₃C₆H₄ | O-t-Bu | 3-MeO | KOH | 80/6 | 4n | 94 | 85 |
| 22 | 2-naphthyl | O-t-Bu | 3-MeO | KOH | 80/6 | 4o | 81 | 78 |
| 23 | 3-pyridyl | O-t-Bu | 3-MeO | KOH | 80/6 | 4p | 90 | 89 |

Now, the superscript ^{b} in Table 1 indicates the isolated yield using silica gel chromatography, the superscript ^{c} indicates the excess mirror image isomer ratio identified using chiral HPLC and superscript ^{d} 3,4-(CH₂O₂) represents the methylene dioxy group.

### Synthesis (Experiment Number 13) of (S)-tert-butyl-3-(4-methoxyphenyl)-3-phenyl propanoate (4f)

bis-Norbornadiene rhodium tetrafluoroborate [Rh(nbd)₂]BF₄ (0.015 mmol, 3 mol%) and (2S, 3S)-(-)-bis(diphenylphosphino) butane {(S,S)-Chiraphos} (0.0165 mmol, 3.3 mol%) were allowed to react for fifteen minutes under a nitrogen atmosphere in 1,4-dioxane (3 ml) and water (0.1 ml) at room temperature to prepare a [Rh(nbd)((S, S)-chiraphos)]BF₄ complex in the system. Subsequently, 0.5 mmol of tert-butyl cinnamate, 0.4 ml of a 1.25 M aqueous potassium hydroxide solution and 1.5 mmol of 4-methoxyphenyl boronic acid were added to the system, and the mixture was stirred for six hours at 50°C. Ether was added to the reaction solution, and the organic layer was washed using a saturated aqueous ammonium chloride solution. Anhydrous magnesium sulfate was added to the organic layer to dry the layer which was then filtered. The solution was concentrated under reduced pressure, and the residue was purified using silica gel chromatography (hexane:ethyl acetate = 30:1 to 15:1) to obtain as the product (S)-tert-butyl-3-(4-methoxyphenyl)-3-phenyl propanoate (287.4 mg, 92%) of Table 1.

The analytical results on the products are shown below. The retention times of the two mirror image isomers were twenty-seven minutes and thirty-three minutes when chiral HPLC (Daicel Chiralpak AD-H) was conducted with hexane:iso-propyl alcohol = 99:1 as the solvent at a flow rate of 0.5 ml/min to examine the products. Based on the results, the enantiomeric excess ratio (%ee) of the product was determined to be 93%ee.
IR(neat): 667, 1139, 1512, 1714, 2838, 2932, 2969, 3005, 3030 cm⁻¹; ¹HNMR (400MHz, CDCl₃) δ 1.29 (s, 3H), 2.94 (d, J = 8.4 Hz, 2H), 3.77 (s, 3H), 4.44 (t, J = 8.4 Hz, 1H), 6.82 (m, 2H), 7.15-7.30 (m, 7H); ¹³CNMR (100 Hz, CDCl₃) δ 28.1, 42.5, 46.8, 55.4, 80.6, 114.0, 126.5, 127.9, 128.6, 128.9, 136.0, 144.1, 158.3, 171.4; exact mass calculated for C₂₀H₂₄O₃: 312.1725, found 312.1722; [α]_{D}²⁶ (c in MeOH): +1.6 (0.96)

Products 4a-4e and 4g-4p were obtained as described below in the manner shown in formula VII using arylboronic acid and the substrates shown in Table 1.
The effect of the base on the reaction was demonstrated as below in the data in Table 1. First, no reaction occurred and the yields were zero when synthesizing (S)-4-(4-methoxyphenyl)-4-phenyl-2-butanone (4a) without using a base in Experiment Number 1 and when using Et₃N or KHCO₃ in Experiment Numbers 2 and 3, respectively. A highly selective reaction proceeded and the yield was excellent when KOH or K₂CO₃ was used as the base in Experiment Numbers 4 and 5, respectively.
Next, the yields were low even when the reaction mixture was stirred for twenty-one hours at a reaction temperature of 65°C when synthesizing (S)-tert-butyl-3-(4-methoxyphenyl)-3-phenyl propanoate (4f) in Experiment Numbers 10, 11 and 12 using Et₃N (yield 40%), K₂CO₃ (yield 76%) and CsCO₃ (yield 83%), respectively. On the other hand, the reaction proceeded at a reaction temperature of 50°C and six hours of stirring with the highest yield when synthesizing 4f using KOH as the base in Experiment Number 13.
Based on the results above, a high yield of a product was obtained using a base in the present invention. Furthermore, KOH and K₂CO₃ were the most preferred bases, and the performance declined in the order of Cs₂CO₃, KHCO₃ and Et₃N. Based on the observation, the most preferred base is an alkali metal hydroxide or a salt of an alkali metal and a weak acid that does not contain hydrogen.

The analytical results for Products 4a-4e and 4g-4p are shown below.
Product: (S)-4-(4-methoxyphenyl)-4-phenyl-2-butanone (4a)
HPLC column, Daicel Chiralcel OD-H: eluent, n-hexane/2-propanol (9:1); flow rate, 0.5 ml/min: t_{R} 18 and 20 min; IR(neat); 700, 838, 1250, 1506, 1605, 1720, 2940, 2960 cm⁻¹; ¹HNMR (400MHz, CDCl₃) δ 2.04 (s, 3H), 3.13 (d, J = 7.6 Hz, 1H), 3.73 (s, 3H), 4.53 (t, J = 7.6 Hz, 1H), 6.95 (d, J = 6.6 Hz, 2H), 7.1 1-7.29 (m, 7H); ¹³CNMR (100 Hz, CDCl₃) δ 30.05, 38.7, 45.2, 49.8, 55.1 113.9, 126.3, 127.5, 128.5, 128.6, 135.9, 144.2, 158.0, 206.8; MS(m/z) 254 (M⁺); [α]_{D}²² (c in CHCl₃): +0.75 (0.60)

Product: (S)-4-(3,4-methylene dioxyphenyl)-4-phenyl-2-butanone (4b)
HPLC column, Daicel Chiralcel OD-H: eluent, n-hexane/2-propanol (9:1): flow rate, 0.5 ml/min: t_{R} 21 and 26 min; IR(neat); 1486, 1230, 1036, 699, 533 cm⁻¹; ¹HNMR (400MHz, CDCl₃) δ 2. 10 (s, 3H), 3.14 (d, J = 7.3 Hz, 2H), 4.52 (t, J = 7.6 Hz, 1H), 5.91 (s, 2H), 6.70-6.74 (m, 3H), 7.17-7.3 1 (m, 5H); ¹³CNMR (400 Hz, CDCl₃) δ 30.6, 45.7, 49.7, 100.9, 108.2, 108.2, 120.5, 126.5, 127.5, 128.6, 137.8, 143.9, 146.0, 147.7, 206.7; MS(m/z) 77(4.8), 152 (22), 211 (100), 225(3.2), 268(44, M⁺); exact mass calculated for C₁₇H₁₆O₃: 268.1099; found 268.1101. [α]_{D}²³ (c in CHCl₃): +1.8 (0.41)

Product: (S)-4-(3-methoxyphenyl)-4-(2-naphthyl)-2-butanone (4c)
HPLC column, Daicel Chiralcel OD-H: eluent, n-hexane/2-propanol (9:1): flow rate, 0.5 ml/min: t_{R} 36 and 41 min; IR(neat); 543, 622, 694, 717, 755, 785, 818, 858, 953, 1041, 1092, 1150, 1258, 1316, 1357, 1434, 1453, 1487, 1582, 1597, 1713 cm⁻¹; ¹HNMR (400MHz, CDCl₃) δ 2. 11 (s, 3H), 3.27 (dd, J = 2.7, 7.5 Hz, 2H), 3.76 (s, 3H), 4.73 (t, J = 7.5 Hz, 1 H), 6.72 (dd, J = 2.5, 8.3 Hz, 1H), 6.80 (m, 1H), 6.86 (d, J = 7.8 Hz, 1H), 7.20 (t, J = 7.8 Hz, 1H), 7.32 (dd, J = 1.8, 8.6, 7.5 Hz, 1H), 7.40-7.46 (m, 2H), 7.67 (s, 1H), 7.76 (d, J = 8.6 Hz, 1H), 7.76-7.79 (m, 2H); ¹³CNMR ( 100 Hz, CDCl₃) δ 30.54, 45.90, 49.23, 54.68, 111.2, 114.0, 120.1, 125.5, 126.0, 126.4, 127.4, 127.6, 128.2, 129.5, 132.1, 133.3, 141.0, 145.2, 160.0, 206.7; MS(m/z) 133 (51), 153 (51), 215 (33), 247 (100), 261 (30), 304 (82, M⁺); exact mass calculated for C₂₁H₂₀O₂: 304.1463; found 604.1475. [α]_{D}²³ (c in CHCl₃): -30 (0.23)

Product: (S)-3-(3-methoxyphenyl)-1,3-diphenylpropan-1-one (4d)
HPLC column, Daicel Chiralcel OD-H: eluent, n-hexane/2-propanol (9:1): flow rate, 0.5 ml/min: t_{R} 22 and 25 min; IR(neat); 553, 691, 725, 750, 872, 922, 983, 1042, 1077, 1149, 1203, 1256, 1361, 1448, 1487, 1582, 1596, 1682 cm⁻¹; ¹HNMR (400MHz, CDCl₃) δ 4.79 (t, J = 7.3 Hz, 1 H), 6.96-6.72 (ddd, J = 0.8, 2.6, 8.2 Hz, 1H), 6.81 (t, J = 2.1 Hz, 1 H), 6.86 (d, J = 7.8 Hz, 1H), 7.16-7.26 (m, 6H), 7.42 (t, J = 7.6 Hz, 2H), 7.55 (tt, J = 1.3, 7.4, 7.5 Hz, 1H), 7.92 (d, J = 7. 1Hz, 2H); ¹³CNMR (100 Hz, CDCl₃) δ 44.48, 45.80, 54.93, 111.2, 113.0, 120.1, 126.3, 127.7, 127.9, 128.4, 129.4, 132.9, 136.9, 143.9, 145.7, 159.5, 197.8; MS(m/z) 77 (45), 103 (86), 105 (100), 133 (23), 165 (22), 197 (42), 211 (69), 316 (63, M⁺); exact mass calculated for C₂₂H₂₀O₂: 316.1463; found 316.1465. [α]_{D}²³ (c in CHCl₃): +7.1 (0.71)

### Product: (S)-3-(3-methoxyphenyl)-1-(4-methoxyphenyl)-3-phenylpropane-1-one (4e)

HPLC column, Daicel Chiralcel OD-H: eluent, n-hexane/2-propanol (4:1); flow rate, 0.5 ml/min: t_{R} 22 and 25 min; IR(neat): 1252, 698, 1597, 1167, 832 cm⁻¹; ¹HNMR (400MHz, CDCl₃) δ 3.67 (d, J = 7.3 Hz, 2H), 3.85 (s, 3H), 4.77 (s, 3H) 4.79 (t, J = 7.3 Hz, 1H), 6.69-6.72 (m, 1 H), 6.80-6.92 (m, 4H), 7.15-7.27 (m, 6H), 7.90-7.94 (m, 2H); ¹³CNMR (400 Hz, CDCl₃) δ 44.3, 46.0,55.1, 55.4, 111.2, 113.7, 114.0, 120.2, 126.3, 127.8, 128.5, 129.5, 130.3, 144.1, 145.9, 159.6, 163.4, 196.4; MS(m/z) 77(12), 107 (5.8), 197 (22), 211 (36), 346 (51, M⁺); exact mass calculated for C₂₃H₂₄O₃: 346.1569; found 346.1565. [α]_{D}²¹ (c in CHCl₃): +11 (0.63)

### Product: (S)-tert-butyl-3-(3-methoxyphenyl)-3-phenyl propanoate (4g)

HPLC column, Daicel Chiralpak AD-H: eluent, n-hexane/2-propanol (99.5:0.5); flow rate, 0.5 ml/min: t_{R} 45 and 51 min; IR(neat): 1725, 1255, 1141, 769, 698 cm⁻¹; ¹HNMR (400MHz, CDCl₃) δ 1.18 (s, 9H), 2.85 (d, J = 8.3 Hz, 2H), 3.61 (s, 3H), 4.35 (t, J = 8.3 Hz, 1H), 6.59-6.62 (m, 1H), 6.69-6.75 (m, 2H), 7.03-7.17 (m, 6H); ¹³CNMR (400 Hz, CDCl₃) δ 27.9, 41.9, 47.3, 54.9, 80.3, 111.4, 113.7, 120.0, 126.3, 127.6, 128.3, 129.3, 143.3, 145.1, 159.5, 170.9; MS (m/z) 197 (100), 210 (30.2), 211 (7.8), 239 (12.7), 312 (6.0, M⁺); exact mass calculated for C₂₀H₂₄O₃: 312.1725; found 312.1719. [α]_{D}²³ (c in CHCl₃): +2.2 (0.52)

### Product: (S)-tert-butyl-3-(3,4-dimethoxyphenyl)-3-phenyl propanoate (4h)

HPLC column, Daicel Chiralpak AD-H: eluent, n-hexane/2-propanol (99:1): flow rate, 0.5 ml/min: t_{R} 68 and 7 min; IR(neat): 1253, 1138, 1028, 700, 511 cm⁻¹; ¹HNMR (400MHz, CDCl₃) δ 1.28 (s, 9H), 2.93 (d, J = 8.3 Hz, 2H), 3.80 (s, 6H), 4.43 (t, J = 8.1 Hz, 1 H), 6.75-6.80 (m, 3H), 7.13-7.27 (m, 5H); ¹³CNMR (400 Hz, CDCl₃) δ 27.8, 42.1, 46.8, 55.5, 80.2, 110.9, 111.1, 119.3, 126.2, 127.4, 128.2, 136.0, 143.6, 147.4, 148.6, 170.8; MS (m/z) 57 (10.1), 227 (100), 269 (5.9), 285 (78.1), 342 (34.6, M⁺); exact mass calculated for C₂₁H₂₆O₄: 342.1831; found 342.1827. [α]_{D}²³ (c in CHCl₃): +0.71 (0.21)

### Product: (S)-tert-butyl-3-(3,4-methylene dioxyphenyl)-3-phenyl propanoate (4i)

HPLC column, Daicel Chiralpak AD-H: eluent, n-hexane/2-propanol (99.5:0.5); flow rate, 0.5 ml/min: t_{R} 61 and 63 min; IR(neat); 1243, 1141, 1037, 699, 515 cm⁻¹; ¹HNMR (400MHz, CDCl₃) δ 1.31 (s, 9H), 2.91-2.93 (m, 2H), 4.41 (t, J = 8.1 Hz, 1 H), 5.87 (s, 2H), 6.70-6.75 (m, 3H), 7.17-7.30 (m, 5H); ¹³CNMR (400 Hz, CDCl₃) δ 27.8, 42.1, 47.0, 80.4, 100.8, 108.0, 108.2, 120.5, 126.4, 127.5, 128.4, 137.5, 143.6, 146.0, 147.6, 170.9; MS (m/z) 57 (6.8), 211 (100), 253 (9.7), 269 (43.9), 326 (15.4, M⁺); exact mass calculated for C₂₀H₂₂O₄: 326.1518; found 326.1519; [α]_{D}²³ (c in CHCl₃): +0.35 (0.43)

### Product: (S)-tert-butyl-3-[4-(N,N-dimethylamino) phenyl]-3-phenyl propanoate (4j)

HPLC column, Daicel Chiralcel OD-H: eluent, n-hexane/2-propanol (99.5:0.5); flow rate, 0.5 ml/min: t_{R} 25 and 30 min; IR(neat); 1716, 1149, 812, 696, 533 cm⁻¹; ¹HNMR (400MHz, CDCl₃) δ 1.27 (s, 9H), 2.88 (s, 6H), 2.92 (d, J = 8.8 Hz, 2H), 4.38 (t, J = 8.3 Hz, 1H), 6.63-6.67 (m, 2H), 7.05-7.27 (m, 7H); ¹³CNMR (400 Hz, CDCl₃) δ 27.8, 40.6, 42.3, 46.5, 80.2, 112.7, 126.1, 127.7, 128.2, 128.3, 131.7, 144.3, 149.2, 171.3; MS (m/z) 57 (6.6), 210 (100), 224 (3.9), 268 (56.0), 325 (29.0, M⁺); exact mass calculated for C₂₁H₂₇NO₂: 325.2042; found 325.2044. [α]_{D}²³ (c in CHCl₃): +3.8 (0.18)

### Product: (S)-tert-butyl-3-(3-methoxyphenyl)-3-(4-methoxyphenyl) propanoate (4k)

HPLC column, Daicel Chiralpak AD-H: eluent, n-hexane/2-propanol (99.5:0.5); flow rate, 0.5 ml/min: t_{R} 76 and 102 min; IR(neat); 1247, 1511, 1176, 1142, 1036 cm⁻¹; ¹HNMR (400MHz, CDCl₃) δ 1.19 (s, 9H), 2.82 (d, J = 8.3 Hz, 2H), 3.62-3.63 (m, 6H), 4.31 (t, J = 8.3 Hz, 1 H), 6.59-6.62 (m, 1H), 6.67-6.73 (m, 4H), 7.04-7.09 (m, 3H); ¹³CNMR (400 Hz, CDCl₃) δ 27.7, 42.1, 46.5, 54.9, 55.0, 80.3, 111.3, 113.7, 119.9, 128.5, 129.3, 135.5, 145.5, 158.0, 159.5, 171.0; MS (m/z) 57 (6.0), 227 (100), 269 (6.4), 285 (54), 342 (8.0, M⁺); exact mass calculated for C₂₁H₂₆O₄: 342.1831; found 342.1826.
[α]_{D}²² (c in CHCl₃): +1.3 (0.41)

### Product: (R)-tert-butyl-(2-methoxyphenyl)-3-(3-methoxyphenyl) propanoate (41)

HPLC column, Daicel Chiralpak AD-H: eluent, n-hexane/2-propanol (99.5:0.5); flow rate, 0.5 ml/min: t_{R} 41 and 52 min; IR(neat); 1490, 1242, 1142, 752, 696 cm⁻¹; ¹HNMR (400MHz, CDCl₃) δ 1.26 (s, 9H), 2.89 (dd, J = 8.7, 15 Hz, 1H), 2.96 (dd, J = 7.8, 15 Hz, 1H), 3.71 (s, 3H); ¹³CNMR (400 Hz, CDCl₃) δ 27.7, 40.4, 40.8, 54.9, 55.2, 80.0, 110.5, 111.1, 113.9, 120.2, 120.3, 127.7, 128.9, 131.7, 144.9, 156.7, 159.3, 171.2; MS (m/z) 57 (21), 227 (29), 241 (9.8), 269 (22), 342 (5.0, M⁺); exact mass calculated for C₂₁H₂₆O₄: 342.1831; found 342.1843. [α]_{D}²² (c in CHCl₃): +21 (0.47)

### Product: (S)-tert-butyl-3-(3-methoxyphenyl)-3-(p-tolyl) propanoate (4m)

HPLC column, Daicel Chiralpak AD-H: eluent, n-hexane/2-propanol (99.5:0.5); flow rate, 0.5 ml/min: t_{R} 40 and 44 min; IR(neat); 1726, 1255, 1142, 779, 697 cm⁻¹; ¹HNMR (400MHz, CDCl₃) δ 1.19 (s, 9H), 2.18 (s, 3H), 2.83 (d, J = 7.8 Hz, 2H), 3.63 (s, 3H), 4.36 (t, J = 8.1 Hz, 1 H), 6.59-6.62 (m, 1 H), 6.69-6.74 (m, 2H), 6.96-7.11 (m, 5H); ¹³CNMR (400 Hz, CDCl₃) δ 20.9, 27.8, 42.0, 46.9, 55.0, 80.3, 111.4, 113.7, 120.0, 127.5, 129.0, 129.3, 135.8, 140.4, 145.4, 159.5, 171.0; MS (m/z) 57 (21), 211 (100), 225 (3.9), 253 (7.9), 269 (16), 326 (4.9, M⁺); exact mass calculated for C₂₁H₂₆O₃: 326.1882; found 326. 1887. [α]_{D}²² (c in CHCl₃): -1.0 (0.53)

### Product: (S)-tert-butyl-3-(3-methoxyphenyl)-3-(4-trifluoromethylphenyl) propanoate (4n)

HPLC column, Daicel Chiralpak AD-H: eluent, n-hexane/2-propanol (99:1): flow rate, 0.5 ml/min: t_{R} 24 and 26 min; IR(neat); 1323, 1257, 1113, 1068, 842 cm⁻¹; ¹HNMR (400MHz, CDCl₃) δ 1.29 (s, 9H), 2.96 (t, J = 8.3 Hz, 2H), 3.74 (s, 3H), 4.52 (t, J = 8.1 Hz, 1 H), 6.73-6.82 (m, 3H), 7.18-7.23 (m, 1 H), 7.36 (d, J = 8.3 Hz, 2H), 7.52 (d, J = 8.3 Hz, 2H); ¹³CNMR (400 Hz, CDCl₃) δ 27.7, 41.5, 47.1, 55.0, 80.7, 111.7, 113.8, 119.9, 122.8, 125.3, 125.3, 128.0, 129.6, 144.2, 147.5, 159.7, 170.5; MS (m/z) 57 (24), 265 (64), 279 (9.9), 307 (18), 323 (7.2), 380 (6.0, M⁺); exact mass calculated for C₂₁H₂₃F₃O₃: 380.1599; found 380.1608. [α]_{D}²² (c in CHCl₃): +2.5 (0.55)

### Product: (S)-tert-Butyl-3-(3-methoxyphenyl)-3-(2-naphthyl) propanoate (4o)

HPLC column, Daicel Chiralpak AD-H: eluent, n-hexane/2-propanol (99:1): flow rate, 0.5 ml/min: t_{R} 3 and 37 min; IR(neat): 1719, 1244, 1140, 758, 712 cm⁻¹; ¹HNMR (400MHz, CDCl₃) δ 1.16 (s, 9H), 2.89-2.99 (m, 2H), 3.60 (s, 3H), 4.53 (t, J = 8.1 Hz, 1H), 6.59-6.61 (m, 1 H), 6.73-6.77 (m, 2H), 7.07 (t, J = 7.8 Hz, 1H), 7.21-7.33 (m, 3H), 7.59-7.68 (m, 4H); ¹³CNMR ( 400 Hz, CDCl₃) δ 27.8, 41.8, 47.3, 55.0, 80.4, 111.5, 113.9, 120.2, 125.4, 125.6, 125.9, 126.5, 127.5, 128.1, 129.4, 132.2, 133.3, 140.8, 145.0, 159.6, 171.0; MS (m/z) 57 (9.0), 247 (100), 261 (3.2), 289 (8.3), 305 (32), 362 (12, M⁺); exact mass calculated for C₂₄H₂₆O₃: 362.1882; found 362.1879. [α]_{D}²² (c in CHCl₃): -18 (0.51)

### Product: (R)-tert-Butyl-3-(3-methoxyphenyl)-3-(3-pyridyl) propanoate (4p)

HPLC column, Daicel Chiralpak AD-H: eluent, n-hexane/2-propanol (99:1): flow rate, 0.5 ml/min: t_{R} 30 and 33 min; IR(neat): 1723, 1257, 1143, 715, 699 cm⁻¹; ¹HNMR (400MHz, CDCl₃) δ 1.21 (s, 9H), 2.84-2.94 (m, 2H), 3.68 (s, 3H), 4.39 (t, J = 8.3 Hz, 1 H), 6.66-6.75 (m, 3H), 7.10-7.21 (m, 2H), 7.45-7.47 (m, 1 H), 8.36-8.37 (m, 1 H), 8.48-8.51 (m, 1H); ¹³CNMR (400 Hz, CDCl₃) δ 27.8, 41.5, 44.9, 55.1, 80.9, 111.8, 113.7, 119.9, 123.3, 129.6, 135.0, 138.8, 143.9, 147.9, 149.3, 159.7, 170.4; MS (m/z) 57 (35), 198 (44), 212 (100), 240 (12), 313 (7.0, M⁺); exact mass calculated for C₁₉H₂₃NO₃: 313.1678; found 313. 1674. [α]_{D}²² (c in CHCl₃): +9.7 (0.52)

### [Example 2]

Methyl(-)-(S)-2-[1-(3,4-methylenedioxyphenyl)-2-(tert-butoxycarbonyl)] ethyl-5-propoxy benzoate (6) was prepared as an optically active β-diaryl electron withdrawing group substituted compound according to the reaction described by the formula VIII below. In formula VIII, the symbol 5 represents a substrate, symbol 3b represents a rhodium complex catalyst and symbol 6 represents a product.

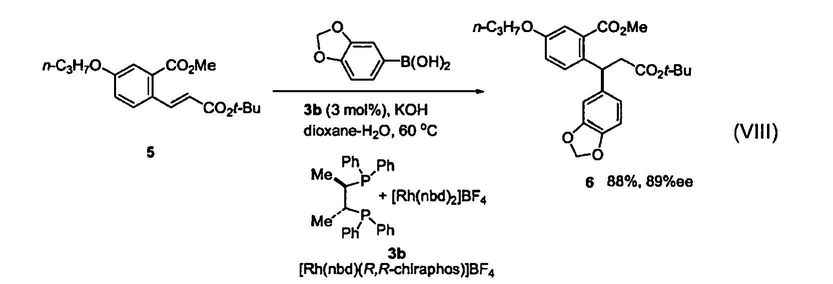

To 1,4-dioxane (10.5 ml)/water (0.7 ml) were added 39.2 mg (3.0 mol%) of [Rh(nbd)₂]BF₃, 49.2 mg (3.3 mol%) of (R,R)-Chiraphos and 1.2 g (3.5 mmol) of (E)-methyl 2-(3-tert-butoxy-3-oxoprop-1-enyl)-5-propoxylbenzoate under a nitrogen atmosphere, and the mixture was stirred for fifteen minutes at room temperature. To the mixture were added 1.25 M (4.2 ml) of an aqueous potassium hydroxide solution and 970 mg (5.3 mmol) of 3,4-(methylenedioxy) phenylboronic acid, and the mixture was stirred for twenty hours at 60°C. The reaction solution was passed through a column packed with silica gel and magnesium sulfate and was washed using a hexane:ethyl acetate solution (1:1). The reaction solution was dried under reduced pressure and was further purified using silica gel column chromatography (hexane:ethyl acetate 20:1) to obtain the compound shown in formula VIII as the product. (1.36 g, 88%, 89%ee).

The analytical results of the product are shown below. Chiral HPLC (Daicel Chiralpak AD-H) was used to evaluate the product with hexane:isopropyl alcohol = 9:1 as the solvent. The retention times for the two mirror image isomers were thirty-three minutes and thirty-six minutes. Based on the results, the enantiometric excess ratio of the product was determined to be 89%ee.
[α]_{D}²³ = -46.5° (c = 0.70, CHCl₃); ¹HNMR (400MHz, CDCl₃) δ 7.30 (d, J = 2.9 Hz, 1 H), 7.22 (d, J = 8.8 Hz, 1 H), 6.97 (dd, J = 8.8, 2.9 Hz, 1H), 6.74-6.86 (m, 3H), 5.87 (s, 2H), 5.31 (t, J = 8.3 Hz, 1H), 3.90 (t, J = 6.3, 6.8 Hz, 2H), 3.87 (s, 3H), 2.87 (dd, J = 3.4, 8.3 Hz, 2H), 1.76 (sextuplet, 6.8, 7.3 Hz, 2H), 1.01 (t, J = 7.3 Hz, 3H); ¹³CNMR(100 MHz, CDCl₃) δ 170.8, 168.0, 157.0, 147.5, 145.7, 137.8, 136.2, 131.0, 129.4, 120.6, 118.3, 115.7, 108.6, 107.8,, 11 100.7, 80.3, 69.6, 52.0, 42.6, 41.3, 27.8, 22.4, 10.4; IR (neat, cm⁻¹): 2971, 1720, 1487, 1436, 1285, 1216, 1143, 1073, 1038, 933, 803; MS (m/z) 57 (12), 253 (20), 267 (18), 295 (65), 309 (41), 327 (40), 340 (54), 354 (100), 367 (27), 386 (68), 442 (11, M⁺); HRMS calculated for C₂₅H₃₀O₇, 442.1922, found 442.1995

### [Example 3]

The individual products 8a-8e described in the formula IX below were prepared as the nitrogen-containing optically active β-diaryl electron withdrawing group substituted compound (8). In the formula, symbol 7 indicates a substrate and symbol 8 indicates a product.

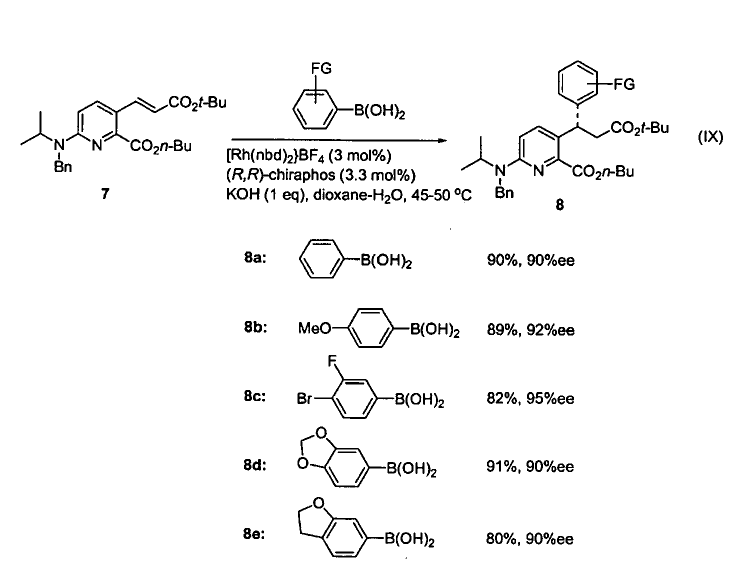

The individual products 8a-8e were obtained as follows. In 1,4-dioxane (2 ml)/water (0.5 ml), 3.0 mol% of [Rh(nbd)₂]BF₄ and 3.3 mol% of (R,R)-Chiraphos were stirred for fifteen minutes under a nitrogen atmosphere at room temperature. To this were added a 1,4-dioxane solution(1.0 mL) of 0.4 mmole of trans-butyl-[6-(benzyl)(isopropyl)amino]-3-(3-tert-butoxy-3-oxo-1-propenyl) picolinate 7, 0.8 mmol of potassium hydroxide and 1.2 mmoles of arylboronic acid, and the reaction solution was stirred for fourteen hours at 50°C. The reaction solution was dried under reduced pressure and was purified using silica gel column chromatography to obtain a product. The enantiomeric excess ratio (%ee) was determined using Chiral HPLC according to the same method described in Examples 1 and 2 above.

The analytical results for the individual products 8a-8e are shown below.

### Product: tert-Butyl-(S)-3-[6-(benzyl)(isopropyl) amino]-2-butoxycarbonyl-3-pyridinyl]-3-phenyl propanoate (8a)

90% Yield; R_{f} =0.61 (n-heptane/ethyl acetate = 2:1); HPLC column, Daicel Chiralcel OD-H: eluent, n-hexane/2-propanol (90:10): flow rate, 0.5 ml/min: t_{R} for 8a, 8.3 min: t_{R} for enantiomer, 9.7 min: 90% ee, [α[_{D}²⁰ = -40.2° (c = CHCl₃): ); ¹HNMR (500MHz, CDCl₃) δ 7.12-7.28 (m, 11 H), 6.31 (d, J = 9.0 Hz, 1 H), 5.04 (sept. J = 6.7 Hz, 1 H), 4.96 (t, J = 8.2 Hz, 1H), 4.48 (s, 2H), 2.87 (d, J = 8.2 Hz, 2H), 1.68-1.76 (m, 2H), 1.48 (m, 2H), 1.25 (s, 9H), 1.16 (d, J = 6.7 Hz, 6H), 0.94 (t, J = 7.3 Hz, 3H); ¹³CNMR (125 MHz, CDCl₃) δ 170.8, 167.6, 156.2, 146.4, 143.1, 139.8, 137.5, 128.4, 128.3, 127.8, 127.6, 126.6, 126.3, 125.7, 109.3, 80.4, 65.1, 46.6, 46.2, 42.0, 41.0, 30.6, 27.9, 27.8, 20.2, 20.1, 19.2, 13.7; IR (KBr, cm⁻¹); 2972, 1726, 1601, 1553; HRMS calculated for C₃₃H₄₃N₂O₄: M⁺ +H), 531.3223. Found 531.3319.

### Product: tert-Butyl-(S)-3-[6-(benzyl)(isopropyl) amino]-2-butoxycarbonyl-3-pyridinyl]-3-(4- methoxyphenyl) propanoate (8b)

89% Yield; R_{f} =0.48 (n-heptane/ethyl acetate = 2:1); HPLC column, Daicel Chiralcel OD-H: eluent, n-hexane/2-propanol (90:10): flow rate, 0.5 ml/min: t_{R} for 8b, 9.7 and 13.7 min: 92% ee, [α]_{D}²⁰ = -28.4° (c = 2.125, CHCl₃); ¹HNMR (500MHz, CDCl₃) δ 7.15-7.28 (m, 8H), 6.78 (d, J = 8.7 Hz, 2H), 6.31 (d, J = 8.9 Hz, 1 H), 5.04 (sept., J = 6.7 Hz, 1 H), 4.89 (t, J = 8.3 Hz, 1 H), 4.48 (s, 2H), 4.30-4.34 (m, 2H), 3.74 (s, 3H), 2.83 (d, J = 8.3 Hz, 2H), 1.72 (m, 2H), 1.38-1.50 (m, 2H), 1.26 (s, 9H), 1.16 (d, J = 6.7 Hz, 6H), 0.94 (t, J = 7.4 Hz, 3H); ¹³CNMR ( 125 MHz, CDCl₃) δ 170.8,167.6,158.0,156.2,146.4, 139.8, 137.4, 135.3, 128.7, 128.4, 126.6, 126.3, 126.0, 113.7, 109.3, 80.4, 65.0, 55.2, 46.6, 46.2, 42.1, 40.3, 30.6, 27.9, 27.8, 20.2, 20.1, 19.2, 13.7; IR (KBr, cm⁻¹) 2972, 1730, 1600, 1553, 1481, 1146, 1077, 1037, 961, 843, 731, 697; HRMS calculated for C₃₄H₄₅N₂O₅ (M⁺ +H), 561.3328. Found 561.3418.

### Product: tert-Butyl-(S)-3-[6-(benzyl)(isopropyl) amino]-2-butoxycarbonyl-3-pyridinyl]-3-(4-bromo-3-fluorophenyl) propanoate (8c)

82% Yield; R_{f} =0.68 (n-heptane/ethyl acetate = 2:1); HPLC column, Daicel Chiralcel OD-H: eluent, n-hexane/2-propanol (90:10): flow rate, 0.5 ml/min: t_{R} for 8c, 8.4 and 11.8 min: 95% ee, [α]_{D}²⁰ = -32.8° (c = 1.145, CHCl₃): ¹HNMR (500MHz, CDCl₃) δ 7.40 (dd, J = 7.5, 8.0 Hz, 1H), 7.18-7.28 (m, 6H), 7.03 (dd, J = 1.8, 9.9 Hz, 1H), 6.94 (dd, J = 1.5, 8.3 Hz, 1H), 6.34 (d, J = 9.0 Hz, 1H), 5.03 (sept., J = 6.7 Hz, 1H), 4.98 (t, J = 8.1 Hz, 1 H), 4.50 (s, 2H), 4.29-4.35 (m, 2H), 2.83 (d, J = 8.1 Hz, 2H), 1.68-1.73 (m, 2H), 1.40-1.47 (m. 2H), 1.28 (s, 9H), 1.18 (dd, J = 1.5, 6.7 Hz, 6H), 0.94 (t, J = 7.4 Hz, 3H); ¹³CNMR ( 125 MHz, CDCl₃) δ 170.3,167.3,156.4,146.3,139.5,137.2,133.2,128.5, 126.7, 126.3, 124.7, 116.1, 115.9, 109.5, 80.8, 65.2, 46.7, 41.5, 40.3, 30.6, 27.8, 20.1, 19.2, 13.7; IR (KBr, cm⁻¹); 2976, 1731, 1600, 1485; HRMS calculated for C₃₃H₄₁BrFN₂O₄ (M⁺ +H), 627.2234. Found, 627.2415.

### Product: tert-Butyl-(S)-3-[6-(benzyl)(isopropyl) amino]-2-butoxycarbonyl-3-pyridinyl]-3-(3,4-dimethylenedioxyphenyl) propanoate (8d)

88% Yield; R_{f} =0.52 (n-heptane/ethyl acetate = 2:1); HPLC column, Daicel Chiralcel OD-H: eluent, n-hexane/2-propanol (90:10): flow rate 0.5 ml/min: t_{R} for 8d, 10.0 and 13.0 min: 90% ee, [α]_{D}²⁰ = -36.5° (c = 1.76, CHCl₃); ¹HNMR (500MHz, CDCl₃) δ 7.18-7.28 (m, 6H), 6.73 (s, 1H), 6.67-6.72 (m, 2H), 6.32 (d, J = 9.0 Hz, 1 H), 5.87 (s, 2H), 5.04 (sept., J = 6.7 Hz, 1H), 4.88 (t, J = 8.2 Hz, 1H), 4.49 (s, 2H), 1.28 (s, 9H), 1.16 (d, J = 6.7, 6H), 0.94 (t, J = 7.4 Hz, 3H); ¹³CNMR ( 125 MHz, CDCl₃) δ 170.0, 167.6, 56.2, 147.6, 146.4, 145.9, 139.7, 137.7, 137.2, 128.4, 126.6, 126.3, 125.7, 120.5, 109.3, 108.6, 107.9, 100.8, 80.5, 65.1, 46.6, 46.2, 42.1, 40.7, 30.7, 27.9, 20.2, 20.1, 19.2, 13.7; IR (KBr, cm⁻¹) 2974, 1731, 1600, 1553, 1484, 1146, 1039, 937, 813, 731, 697; HRMS calculated for C₃₄H₄₃N₂O₆ (M⁺ +H), 575.3121. Found, 575.3218.

### Product: tert-Butyl-(S)-3-[6-(benzyl)(isopropyl) amino]-2-butoxycarbonyl-3-pyridinyl]-3-(2,3-dihydro-1-benzofuran-6-yl) propanoate (8e)

80% Yield; R_{f} =0.55 (n-heptane/ethyl acetate = 2:1); HPLC column, Daicel Chiralcel OD-H: eluent, n-hexane/2-propanol (90:10): flow rate, 0.5 ml/min: t_{R} for 8e, 11.2 and 16.9 min: 90% ee, [α]_{D}²⁰ = -39.7° (c = 1.06, CHCl₃); ¹HNMR (500MHz, CDCl₃) δ 7.18-7.28 (m, 6H), 7.04 (d, J = 7.6 Hz, 1 H), 6.74 (d, J = 7.6 Hz, 1 H), 6.67 (s, 1 H), 6.31 (d, J = 9.0 Hz, 1H), 5.04 (sept., 6.6 Hz, 1H), 4.91 (t, J = 8.2 Hz, 1 H), 4.50 (t, J = 8.6, 2H), 4.49 (s, 2H), 4.30-4.35 (m, 2H), 3.11 (t, J = 8.6 Hz, 2H), 2.82 (d, J = 8.1 Hz, 2H), 1.69-1.74 (m, 2H), 1.27 (s, 9H), 1.16 (d, J = 6.6 Hz, 6H), 0.94 (t, J = 7.4 Hz, 3H); ¹³CNMR (125 MHz, CDCl₃) δ 170.8, 167.5, 160.3, 156.2, 146.4, 143.7, 139.8, 137.4, 128.4, 126.6, 126.3, 125.8, 124.8, 124.5, 120.0, 109.3, 108.7, 80.4, 71.2, 65.0, 46.6, 46.2, 42.0, 40.8, 30.7, 29.5, 27.9, 20.2, 20.1, 19.2, 13.7: IR (KBr, cm⁻¹) 2975, 1730, 1599, 1481, 1147, 1078, 989, 947, 813, 759, 697; HRMS calculated for C₃₅H₄₅N₂O₅: (M⁺ +H) 573.3328. Found, 573.3412.

(R)-tert-Butyl-3-(3-(cyclopentyloxy)-4-methoxyphenyl)-3-phenyl propanoate (10) was prepared according to the reaction described in formula X below as the optically active β-diaryl electron withdrawing group substituted compound. In formula X, symbol 9 represents a substrate and symbol 10 represents a product.

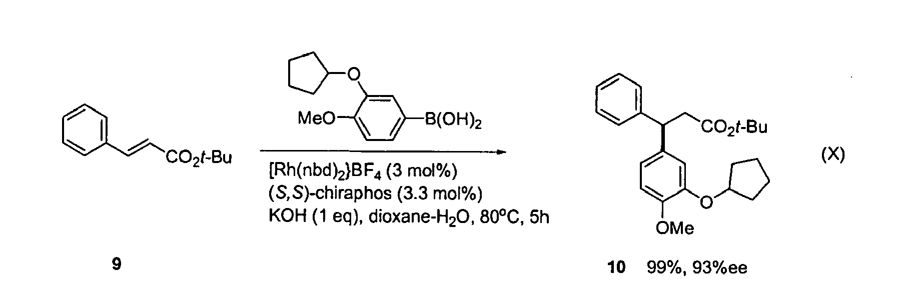

In 3 ml of 1,4-dioxane and 0.1 ml of water, 0.015 mmole (3 mol%) of bis-norbornadiene rhodium tetrafluoroborate [Rh(nbd)₂]BF₄ and 0.0165 mmole (3.3 mol%) of (2S, 3S)-(-)-bis (diphenylphosphino) butane [(S,S)-Chiraphos] were allowed to react for fifteen minutes at room temperature under a nitrogen atmosphere to prepare a [Rh(nbd)(S,S)-Chiraphos] BF₄ complex in the solution. Subsequently, 0.5 mmol of tert-butyl cinnamate, 0.4 ml of 1.25 M aqueous potassium hydroxide solution and 1.5 mmoles of 3-(cyclopentyloxy)-4-methoxyphenyl boronic acid were added to the solution, and the mixture was stirred for five hours at 80°C. Ether was added to the reaction solution, and the organic layer was washed using a saturated aqueous ammonium chloride solution. Anhydrous magnesium sulfate was added to dry the organic layer after which it was filtered and concentrated under reduced pressure, and the residue was purified using silica gel chromatography to obtain (R)-tert-butyl-3-[3-(cyclopentyloxy)-4-methoxyphenyl]-3-phenyl propanoate as product 10.

The analytical results for the product are shown below. Chiral HPLC (Daicel Chiralpak 1A) was used, and the product was evaluated using hexane:isopropyl alcohol:dichloromethane = 100:1:5 as the solvent. The retention times for the two mirror image isomers were ten minutes and eleven minutes. Based on the results, the enantiomeric excess ratio (%ee) of the product was determined to be 93%ee.
[α]_{D}²³ (c in CHCl₃): +0.99 (0.45); ¹HNMR (400MHz, CDCl₃) δ 7.28-7.21 (m, 4H), 7.16 (t, J = 6.8 Hz, 1 H), 6.76-6.74 (m, 3H), 4.71-4.68 (m, 1 H), 4.40 (t, J = 8.3 Hz, 1 H), 3.78 (s, 3H), 2.93 (d, J = 8.3 Hz, 2H), 1.88-1.71 (m, 6H), 1.59-1.54 (m, 2H), 1.27 (S, 9H); ¹³CNMR (100 MHz, CDCl₃) δ 23.9, 27.7, 32.68, 32.69, 42.2, 46.8, 55.9, 80.2, 80.3, 111.7, 114.8, 119.6, 126.2, 127.5, 128.3, 126.0, 143.8, 147.3, 171.1; MS (m/z): 213 (100), 229 (19), 272 (81), 339 (30), 396 (28, M⁺); exact mass calculated for C₂₅H₃₂O₄: 396.2300; found 396.2311.

(S)-tert-Butyl-3-(2-(benzyloxy)-5-methylphenyl)-3-phenyl propanoate (12) was prepared as the optically active β-diaryl electron withdrawing group substituted compound according to the reaction described by formula XI shown below. In formula XI, symbol 11 represents the substrate and symbol 12 represents the product.

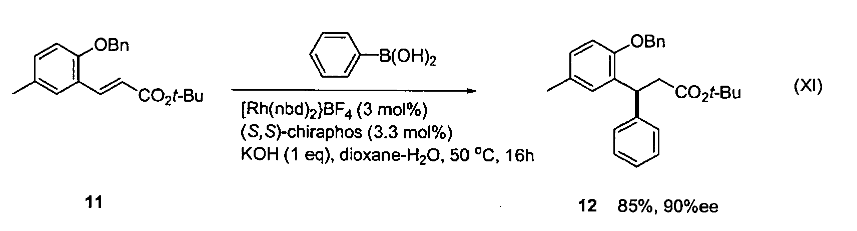

In 3 ml of 1,4-dioxane and 0.1 ml of water, 0.015 mmole (3 mol%) of bis-norbornadiene rhodium tetrafluoro borate [Rh(nbd)₂]BF₄ and 0.0165 mmol (3.3 mol%) of (2S, 3S)-(-)-bis (diphenylphosphino) butane {(S,S)-Chiraphos} were allowed to react for fifteen minutes at room temperature under a nitrogen atmosphere to prepare a [Rh(nbd)(S,S)-Chiraphos] BF₄ complex in the solution. Subsequently, 0.5 mmol of (E)-tert-butyl3-[2-(benzyloxy)-5-methylphenyl] acrylate, 0.4 ml of 1.25 M aqueous potassium hydroxide solution and 1.5 mmoles of phenyl boronic acid were added to the solution, and the mixture was stirred for sixteen hours at 50°C. Ether was added to the reaction solution, and the organic layer was washed using a saturated aqueous ammonium chloride solution. Anhydrous magnesium sulfate was added to dry the organic layer which was filtered after drying and concentrated under reduced pressure, and the residue was purified using silica gel chromatography to obtain (S)-tert-butyl-3-(2-(benzyloxy)-5-methylphenyl)-3-phenyl propanoate as the product.

The analytical results for the product are shown below. Compound 12 was converted into a n-butyl ester, and the product was evaluated using Chiral HPLC (Daicel Chiralpak AD-H) with hexane:isopropyl alcohol = 99.5:0.5 as the solvent. The retention times for the two mirror image isomers were 31.6 minutes and 38.0 minutes. Based on the results, the enantiomeric excess ratio (%ee) of the product was determined to be 90%ee.
[α]_{D}²³ (c in CHCl₃): +11.1 (0.70); ¹HNMR (400MHz, CDCl₃) δ 7.35-7.12 (m, 10H), 7.01 (s, 1H), 6.93 (dd, J = 1.5, 8.3 Hz, 1 H), 6.75 (d, J = 8.3 Hz, 1 H), 5.01 (d, J = 4.4 Hz, 2H), 4.90 (t, J = 8.3 Hz, 1 H), 3.00 (dd, J = 7.8, 15.1 Hz, 1 H), 2.90 (dd, J = 8.8, 15.1 Hz, 1H), 1.25 (S, 9H); ¹³CNMR ( 100 MHz, CDCl₃) δ 20.7, 27.8, 40.94, 40.99, 69.9, 80.1, 111.8, 126.0, 127.1, 127.5, 127.6, 128.0, 128.1, 128.3, 128.6, 129.6, 131.9, 137.3, 143.3, 153.8, 171.3: MS (m/z): 91 (100), 195 (15), 238 (19), 255 (38), 328 (10), 346 (12), 402 (4, M⁺); exact mass calculated for C₂₇H₃₀O₃: 403.2195; found 402. 2191.

As described above, β-diaryl electron withdrawing group substituted compound can be prepared in high yields and excellent selectivity according to the production method of the embodiment of the present invention. Nitrogen-containing optically active β-diaryl electron withdrawing group substituted compound and optically active β-diaryl esters that were previously difficult to prepare with excellent selectivity were particularly obtained. Furthermore, compounds of formula (VIII) and compounds 8e of formula (IX) that are direct intermediates for endoserine receptor antagonist agents, intermediates (formula X) of PDE4 inhibitor, CPD-840, the intermediate for tolterodine used in a muscarinic receptor antagonist (formula XI) and the like can be obtained. Also, pharmaceutical products may be prepared from these intermediates using an existing method while maintaining high optical purity.

## Claims

1. A method for manufacturing a β-diaryl electron withdrawing group substituted compound wherein an electron withdrawing group substituted β-arylolefin derivative represented by general formula (I) 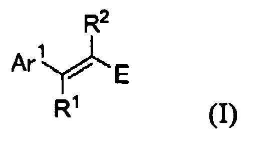 (in the formula, Ar¹ represents a substituted or unsubstituted aryl group, R¹ and R² individually may be identical or different from each other and represent hydrogen or alkyl groups and E represents a formyl, acyl, carboxyl, alkoxycarbonyl, carbamoyl, cyano or nitro group) is allowed to react with
An arylboronic acid or its derivative represented by general formula (II) Ar²-BXₘMₙ or an arylboronic acid anhydride represented by general formula (III) 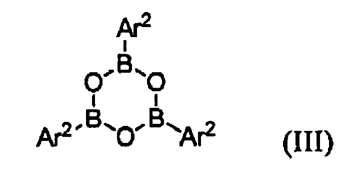 (in formulae II and III, Ar² represents a substituted or unsubstituted aryl group, X indicates a hydroxyl group, alkoxy group or fluorine atom, m is an integer from one to three, M is an alkali metal and n is an integer, zero or one)
in the presence of a rhodium complex catalyst represented by general formula (IV) RhYₒL¹ₚ (Chiraphos)_{q} (in the formula, Y represents ClO₄, BF₄, PF₆, SbF₆, OTf, halogen atom, hydroxyl group, alkoxy group or acyloxy group, L¹ represents an organic ligand, o, p, and q each represent an integer from zero to six) to produce an optically active β-diary electron withdrawing group substituted compound represented by general formula (V) 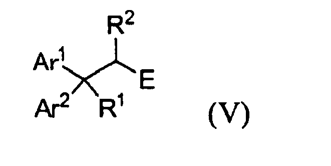 (in formula V, Ar¹, Ar², R¹, R² and E are identical to the substituents used in formulae I-IV).

2. The method for manufacturing a β-diaryl electron withdrawing group substituted compound described in Claim (1) wherein a base is added to the reaction system.

3. The method for manufacturing a β-diaryl electron withdrawing group substituted compound described in Claim (2) wherein the base being one or at least two selected from a group of amines, alkali metal hydroxides or their salts of weak acids, alkaline earth metal hydroxides or their salts of weak acids and quaternary ammonium hydroxides or their salts of weak acids.

4. The method for manufacturing a β-diaryl electron withdrawing group substituted compound described in any one of Claims (1) through (3) wherein the optically active β-diaryl electron withdrawing group substituted compound being an optically active β-diaryl electron withdrawing group substituted compound containing nitrogen or an optically active β-diaryl ester.
